# EUROPEAN PATENT APPLICATION

(11) **EP 3 037 094 A1**
(43) Date of publication of application: **29.06.2016**
(21) Application number: 14460134.1
(22) Date of filing: 23.12.2014
(51) Int. Cl.: A61K 31/335, A61P 27/14

(54) **Ophthalmic pharmaceutical composition**

(71) Applicant: Poifa Warszawa SA, 01-207 Warszawa (PL)
(72) Inventor: Grajewska-Wozniak, Bogna, 01-207 Warszawa (PL)
(74) Representative: Chmurzynski, Sedzimir Lech

(57) **Abstract**

The present invention relates to an aqueous topical ophthalmic pharmaceutical composition comprising olopatatadine or pharmaceutically acceptable salts thereof, wherein the composition does not comprise a preservative.

## Description

### Field of the invention

The present invention relates to an aqueous topical ophthalmic pharmaceutical composition comprising olopatadine or pharmaceutically acceptable salt thereof.

### Background of the invention

Olopatadine is a selective histamine H1 antagonist that binds to the histamine H1 receptor. This blocks the action of endogenous histamine, which subsequently leads to temporary relief of the negative symptoms brought on by histamine. Olopatadine is devoid of effects on alpha-adrenergic, dopamine and muscarinic type 1 and 2 receptors.

Olopatadine is a known anti-allergy drug. It is commercially available in Europe under the brand name Opatanol^{®} marketed by Alcon Laboratories Ltd. The drug is indicated for the treatment of ocular signs and symptoms of seasonal allergic conjunctivitis. The marketed product composition contains olopatadine hydrochloride, benzalkonium chloride, sodium chloride, disodium phosphate dodecahydrate, hydrochloric acid and/or sodium hydroxide (to adjust pH) and purified water.

It has been established that the continuous use of ocular medicines is linked to numerous side effects on the eye. These side effects are related to concentration and type of the medicament, the duration of the treatment and type of preservative used in the formulation. Possible side effects associated with the administration of olopatadine include stinging, irritation, itching, redness, burning or dryness of the eyes.

A variety of different preservatives have been used in ophthalmic preparations. Examples of preservatives used in ophthalmological compositions are summarized in Table 1. It has been recognized that such preservatives may also cause many side effects including irritation of the eye caused by damage of the epithelial cells.

**Table 1. Examples of preservatives used in ophthalmic formulations**

| Compound class | Example |
|---|---|
| Quaternary ammoniums | Benzalkonium chloride, Polyquaternium-1 |
| Mercurials | Thimerosal, Phenyl mercuric nitrate, phenyl mercuric acetate |
| Alcohols | Chlorbutanol, Benzyl alcohol |
| Carboxylic acid | Sorbic acid |
| Phenols | Methyl/propyl paraben |
| Amidines | Chlorhexidine |
| Other | Disodium EDTA |

The most common preservative used in ocular formulations is benzalkonium chloride (BAC). The - compound is a cationic-surfactant having anti-bacterial properties. Benzalkonium chloride has been found to increase the corneal penetration of a number of drugs by emulsification and disruption of cell junctions in the corneal epithelium. BAC works by adhering to the cell membrane of microorganism and increases their permeability which leads to cell lysis. It has been recognized that mammalian cells are unable to neutralize BAC and corneal epithelium is damaged by its entrance. This induces cytotoxic damage to conjunctival cells and stroma. In vitro and in vivo studies demonstrates that BAC can induce toxic and inflammatory effects on the ocular surface.

Topical delivery of drugs to the ocular tissue is affected by a complex of factors. Any change in formulation may have an impact on corneal penetration and ocular absorption.

Olopatadine is a lipophilic compound and therefore does not pass easily across the biological barriers like cornea. Ocular bioavailability can be enhanced by using preservatives which act also as penetration enhancers, e.g. benzalkonium chloride (BAC). Unfortunately, most of such preservatives/penetration enhancers while promoting permeation of drugs may also damage the cornea.

Many attempts have been made to provide ophthalmic compositions of olopatadine having required permeability and adequate ocular absorption of the drug and at the same time reduced occurrence of side effects associated with the use of preservatives, in particular BAC.

International patent application WO01/54687 describes a formulation comprising olopatadine and polymeric quaternary ammonium compound as a preservative. The formulation according to the invention does not contain benzalkonium chloride.

In addition, patent application US2005/0209312A discloses a topical formulation comprising a carboxylic acid derivative, e.g. olopatadine and a gentle preservative. The preservative according to the invention is a stabilized chlorine dioxide.

Moreover, international patent application WO2008/036847 describes an ophthalmic composition comprising olopatadine and zinc based preservative system where the concentration of the zinc ions is from 0.04 to 0.9 mM. The concentration of anionic species in the composition is limited to less than 15 mM to improve composition's antimicrobial activity. In addition, the composition may also comprise a borate or a borate/polyol complex.

Thus there is still a need to provide a topical composition of olopatadine or pharmaceutically acceptable salt thereof having desirable bioavailability and showing no toxicological effects associated with preservatives.

### Description of the invention

Accordingly, the present invention provides an aqueous topical ophthalmic pharmaceutical composition comprising olopatadine or pharmaceutically acceptable salt thereof, wherein the composition does not comprise a preservative. Surprisingly, it has been discovered that such composition demonstrates similar transcorneal permeation and provides similar in vitro ocular availability through HCE-T model as formulation preserved with benzalkonium chloride (BAC) and at the same time demonstrates decrease in damage of the epithelial cells.

Olopatadine is preferably used in composition of the present invention in the form of a salt. Examples of pharmaceutically salts of olopatadine include inorganic salts such as hydrochloride, hydrobromide, sulfate, phosphate; organic salts such as acetate, maleate, furoate, tartrate and citrate; metal salts such as sodium salt, magnesium salt, potassium salt, calcium salt, aluminium salt and zinc salt; organic amine addition salts such as triethylamine addition salt, morpholine addition salt and piperidine addition salt. Preferably, olopatadine is used in the present invention in the form of hydrochloride salt.

Olopatadine or pharmaceutically acceptable salt thereof is preferably present in the compostion according to the present invention in an amount of 0.01 to 5% (w/v) wherein the used amount corresponds to olopatadine free base. In a preferred embodiment of the present invention, olopatadine or pharmaceutically acceptable salt thereof is present in the compostion in an amount of 0.01 to 0.2 % (w/v) wherein the used amount corresponds to olopatadine free base. In the most preferred embodiment of the present invention olopatadine or pharmaceutically acceptable salt thereof is used in the composition in an amount of 0.1 % (w/v) wherein the used amount corresponds to olopatadine free base.

Pharmaceutical composition of the present invention preferably comprises a surfactant not acting as a preservative, more preferably a nonionic surfactant. Examples of surfactants that may be used in ophthalmic composition of the present invention include oxyethylated tertiary octylphenol formaldehyde polymer known as tyloxapol, polyethohylated Castrol oil derivatives, polyethoxylene sorbitan fatty acid esters and mixtures thereof. In a preferred embodiment of the present invention surfactant is polyethoxylene sorbitan fatty acid esters, most preferably polysorbate 80. A surfactant may be used in ophthalmic composition of the invention in an amount of more than 0.01 % (w/v), preferably from 0.05 % to 3 % (w/v), more preferably from 0.1 % to 1 % (w/v), most preferably from 0.1 to 0.5 % (w/v).

Pharmaceutical composition of the present invention preferably comprises at least one further excipient selected from the group consisting of buffering agents, pH adjusting agents, viscosity modifying agents and tonicity agents, preferably buffering agents, tonicity agents and pH adjusting agents.

Ophthalmic composition of the present invention may include an effective amount of a buffering agent. Examples of buffering agents that may be used in ophthalmic composition of the present invention include phosphates, citrates, acetates and mixtures thereof. In a preferred embodiment of the present invention a buffering agent is selected form citric acid, phosphoric acid, sodium or potassium phosphate, dibasic sodium phosphate, sodium or potassium acetate and mixture thereof, more preferably a buffering agent is dibasic sodium phosphate. The most preferred buffering agent used in ophthalmic composition of the present invention is disodium phosphate dodecahydrate. A buffering agent may be used in ophthalmic composition of the present invention in an amount of 0.5 % to 3 % (w/v), preferably 1 % to 1.5 % (w/v), most preferably 1.26 % (w/v).

Ophthalmic composition of the present invention may include an effective amount of a viscosity modifying agent. Examples of viscosity modifying agents that may be used in ophthalmic composition of the present invention include cellulosic ethers such as hydroxypropyl methyl cellulose, hydroxyethyl cellulose, hydroxypropyl cellulose, methyl cellulose, carboxymethyl cellulose; carbomers such as polyvinyl alcohol, polyvinyl pyrrolidone, polyacryclic acid; polysaccharides and mixture thereof.

Ophthalmic composition of the present invention may include an effective amount of a tonicity agent. Examples of tonicity agents that may be used in ophthalmic composition of the present invention include sodium chloride, mannitol, glycerin, sorbitol and mixture thereof. In a preferred embodiment of the present invention a tonicity agent is sodium chloride. A tonicity agent used in ophthalmic composition of the present invention may be used in an amount of 0.1 to 3 % (w/v), preferably 0.4 to 1 % (w/v), most preferably 0.6 % (w/v).

Ophthalmic composition of the present invention may comprise a pH adjusting agent, preferably in an amount sufficient to obtain a composition having a pH of 4 to 8. Preferably, ophthalmic composition of the present invention has a pH of 6 to 7.5, more preferably 7. Strong acids such as hydrochloride acid and strong base such as sodium hydroxide may be used to adjust pH.

In one embodiment ophthalmic composition of the present invention consists of olopatadine or pharmaceutically acceptable salts thereof, a surfactant, a tonicity agent, a buffering agent and a pH adjusting agent and none of the used components is known to act as a preservative.

Ophthalmic composition of the present invention can be packed in suitable containers. In a preferred embodiment of the present invention the composition is packed in a low-density polyethylene container. Preferably, the used container is an aseptic container design to deliver not preserved ophthalmic compositions in liquid form.

Furthermore, the present invention relates to a process for the preparation of the ophthalmic composition as described above. The ophthalmic composition of the present invention may be prepared by the process which comprises the following steps:
a). Dissolving at least one agent selected from the group consisting of a surfactant, a buffering agent, a pH adjusting agent, a viscosity modifying agent and a tonicity agent in a water for injection, preferably a surfactant, a buffering agent, a pH adjusting agent and a tonicity agent in a water for injection, wherein none of the used components is known to act as a preservative;
b). Adding olopatadine or pharmaceutically acceptable salts thereof to the solution prepared in step (a);
c). Preferably adjusting pH of the solution as obtained in step (b) with a pH adjusting agent;
d). Preferably performing final adjustment of volume of the solution as obtained in step (c) with water, preferably water for injection;
e). Preferably filtering of the solution as obtained in step (d), preferably through a 0.22 µm membrane;
f). Preferably transferring and aseptic filling of the solution as obtained in step (e) to sterile containers.

In a preferred embodiment the ophthalmic composition of the present invention is prepared by the process which comprises the following steps:
a). Dissolving a buffering agent in a water for injection and further also dissolving at least one agent selected from the group consisting of a surfactant, a pH adjusting agent, a viscosity modifying agent and a tonicity agent in a water for injection, preferably a surfactant, a pH adjusting agent and a tonicity agent in a water for injection, wherein none of the used components is known to act as a preservative;
b). Adding olopatadine or pharmaceutically acceptable salts thereof to the solution prepared in step (a);
c). Preferably adjusting pH of the solution as obtained in step (b) with a pH adjusting agent;
d). Preferably performing final adjustment of volume of the solution as obtained in step (c) with water, preferably water for injection;
e). Preferably filtering of the solution as obtained in step (d), preferably through 0.22 µm membrane;
f). Preferably transferring and aseptic filling of the solution as obtained in step (e) to sterile containers.

The following examples are provided to illustrate different aspects and embodiments of the present invention. These examples are not intended in any way to limit the disclosed invention. The following abbreviations and definitions have been used in the examples:

**BSS** - Balanced Salt Solution

**Cell culture medium (nutrient medium)** - Dulbecco's Modified Eagle's Medium/Nutrient F-12 Ham is 1:1 mixture of DMEM and Ham's F-12 supplemented with 5% heat activated FBS (Fetal bovine serum), 10 ng/ml hEGF (human epidermal Growth Factor), 10 µg/ml insulin, 0.5% DMSO, 2mM L-Glutamine and antibiotics (Penicilin and Streptomycin).

**HCE-T** (SV40-Adeno vector transformed cornea cells obtained from Riken Cell Bank, Cat no RCB2280) - adherent non-tumorigenic human corneal epithelial cell line. HCE-T line was formed by transfection of human corneal epithelial cells from 47-old female donor with recombinant SV40-adenovirus.

**Transepithelial Electrical resistance (TEER) measurement -** assessment of barrier integrity of corneal epithelium in vitro model, which reflects mainly the resistance across epithelial tight junctions. TEER measurements are taken using Millicell-ERS ohmmer.

### Example 1

| Ingredient | mg/ml |
|---|---|
| Olopatadine hydrochloride | 1.11* |
| Disodium phosphate dodecahydrate | 12.61 |
| Sodium chloride | 5.0 |
| HCl/NaOH | 8 mM |
| Purified water | Ad to 1 ml |

| | |
|---|---|
| *1.11 mg/ml Olopatadine hydrochloride is equivalent to 1 mg/ml of Olopatadine free base | |

The required amount of disodium phosphate dodecahydrate was dissolved in water for injection and sodium chloride was added. The required amount of olopatadine hydrochloride was added to the obtained solution. The pH of the solution was adjusted by using HCl or NaOH to 7 and distilled water was added to bring the final volume up to 1 ml.

### Example 2

| Ingredient | mg/ml |
|---|---|
| Olopatadine hydrochloride* | 1.11* |
| Polysorbate 80 | 5.0 |
| Disodium phosphate dodecahydrate | 12.61 |
| Sodium chloride | 6.0 |
| HCl/NaOH | 8 mM |
| Purified water | Ad to 1 ml |

| | |
|---|---|
| *1.11 mg/ml Olopatadine hydrochloride is equivalent to 1 mg/ml of Olopatadine free base | |

The required amount of disodium phosphate dodecahydrate was dissolved in water for injection and sodium chloride and polysorbate 80 was added to the obtained solution. The required amount of olopatadine hydrochloride was added to the solution. The pH of the solution was adjusted by using HCl or NaOH to 7 and distilled water was added to bring the final volume up to 1 ml.

### Example 2A

| Ingredient | mg/ml |
|---|---|
| Olopatadine hydrochloride* | 1.11* |
| Polysorbate 80 | 1.0 |
| Disodium phosphate dodecahydrate | 12.61 |
| Sodium chloride | 6.0 |
| HCl/NaOH | 8mM |
| Purified water | Ad to 1 ml |

| | |
|---|---|
| *1.11 mg/ml Olopatadine hydrochloride is equivalent to 1 mg/ml of Olopatadine free base | |

The required amount of disodium phosphate dodecahydrate was dissolved in water for injection and sodium chloride and polysorbate 80 was added to the obtained solution. The required amount of olopatadine hydrochloride was added to the solution. The pH of the solution was adjusted by using HCl or NaOH to 7 and distilled water was added to bring the final volume up to 1 ml.

### Example 3

### Comparative example of a composition comprising a benzalkonium chloride as a preservative.

| Ingredient | mg/ml |
|---|---|
| Olopatadine hydrochloride* | 1.11* |
| Disodium phosphate dodecahydrate | 12.61 |
| Sodium chloride | 6.0 |
| Benzalkonium chloride | 0.1 |
| HCl/NaOH | 8 mM |
| Purified water | Ad to 1 ml |

| | |
|---|---|
| *1.11 mg/ml Olopatadine hydrochloride is equivalent to 1 mg/ml of Olopatadine free base | |

The required amount of disodium phosphate dodecahydrate was dissolved in water for injection. The required amount of sodium chloride, polysorbate 80 and benzalkonium chloride was added to the obtained solution. Olopatadine hydrochloride was added to the obtained solution. The pH of the solution was adjusted by using HCl or NaOH to 7 and distilled water was added to bring the final volume up to 1 ml.

### Example 4

Human epithelial cell culture model was constructed to evaluate the permeation of the compositions. In the present study the corneal permeation was evaluated using HCE-T model.

### Construction of corneal epithelium in vitro model

1. The Millicell insert membranes were covered with type I rat tail collagen.
2. The plate was incubated at 37°C for approximately 4h.
3. After incubation the remaining collagen solution was removed and each insert was washed twice with 400 µL DMEM/Ham's F12 mixture (1:1).
4. HCE-T cells were plated at a seeding density of 1x10⁵ cells per cm² in 400 µl of the cell culture medium.
5. The cell culture plate was incubated at 37°C with 5% CO₂ and 95% humidity.
6. Integrity of cell monolayer was evaluated by TEER values measurement at approximately 2-3 intervals. Culture medium was replaced after each TEER measurement.
7. After reaching cell confluence (TEER ≥ 200 Ωcm²) culture medium from apical part was removed and cells were exposed at air - liquid interface (ALI) condition.
8. The HCE-T model was cultivated at the ALI for 7 days (37°C with 5% CO₂ and 95% humidity), with TEER measurement and medium replacement 3 times per week.

### Example 5

### Permeability study performance

1. After the desired cell growth period, the Millicell^{®} - 24 cell culture plate was removed from the incubator and TEER value for each insert was determined.
2. The cornea epithelium model was raised twice with sterile BSS/10 mM Hepes to remove protein and residual nutrient medium. After washing, the buffer was removed from the filter plate and feeder tray.
3. The filter plate was transferred to a 24-well transport analysis plate.
4. To determine the rate of the drug transport in the apical basolateral direction, 400 µl ophthalmic solutions, i.e. composition of Example 1, composition of Example 2 and composition of Example 3 were added to filter wells. The wells of the receiver plate were filled in with 800 µL BSS/10 mM Hepes.
5. The plate was incubated up to 4 hours at RT with orbital shaking at 100 rpm.
6. At the end of the incubation, fixed volumes of solutions from the apical and basolateral chambers were transferred to the glass vials for HPLC analysis.
7. TEER values were measured.

### Example 6

Permeation studies were performed using olopatadine hydrochloride tested solutions, i.e. composition of Example 1, composition of Example 2 and composition of comparative Example 3.

Corneal epithelium in vitro model was constructed according to the protocol described in Example 4. HCE-T cells were plated on a Millicell^{®} collagen-coated PCV filters having a pore-size of 0.4 µm at seeding density of 1x10⁵ cells per cm². The HCE-T cells were first cultivated, submerged in medium for 9 days (TEER > 200 Ωcm²) and then cultivated at the ALI for 7 days more.

The compositions of Examples 1 to 3 were evaluated to determine the permeation on the HCE-T in vitro model. Tested olopatadine solutions, i.e. composition of Example 1 and composition of Example 2 demonstrated similar permeability on human corneal epithelium in vitro model (HCE-T) to that of composition of comparative Example 3. The results are shown in Table 4.

**Table 4. Permeability through human corneal epithelium in vitro model (HCE-T)**

| **Formulation** | **Permeation coefficient cm/s** |
|---|---|
| Composition of Example 1 | 2.28 x 10⁻⁶ |
| Composition of Example 2 | 3.03 x 10⁻⁶ |
| Composition of comparative Example 3 | 3.42 x 10⁻⁶ |

In addition, barrier integrity of HCE-T model was assessed by measurements of TEER values. Constructed HCE-T model demonstrated tight epithelial barrier functions with TEER initial value of approximately 300 Ωcm². Olopatadine formulation preserved with 0.01 % w/v benzalkonium chloride, i.e. composition of comparative Example 3 caused a significant decrease in the HCE-T model TEER initial value, i.e. approximately by 65 %. The tested not preserved olopatadine solutions, i.e. composition of Example 1 and composition of Example 2 reduced the TEER initial value by approximately 35 % and approximately 41 %, respectively. The above results demonstrate that using preservatives has a significant impact on integrity of the human corneal epithelial cells. Composition preserved with benzalkonium chloride, i.e. composition of comparative Example 3, decreased barrier integrity of the epithelial cells by much higher value than compositions that do not comprise preservatives, i.e. composition of Example 1 and composition of Example 2. Thus, it is to be noted that compositions according to the present invention are much more favorable as they produced similar permeability to the preserved compositions and at the same time showed reduction in damage of the epithelial cells.

## Claims

1. An aqueous topical ophthalmic pharmaceutical composition comprising olopatatadine or pharmaceutically acceptable salts thereof, wherein the composition does not comprise a preservative.

2. The composition according to claim 1, wherein the olopatadine is olopatadine hydrochloride.

3. The composition according to any of the preceding claims, wherein olopatadine or pharmaceutically acceptable salts thereof is present in the composition in an amount of 0.01 to 5 % (w/v), preferably 0.01 to 0.2 % (w/v), most preferably 0.1 % (w/v) and wherein the used amount corresponds to olopatadine free base

4. The composition according to any of the preceding claims, wherein the composition comprises a surfactant, preferably a nonionic surfactant.

5. The composition according to claim 4, wherein the surfactant is selected from the group consisting of oxyethylated tertiary octylphenol formaldehyde polymer, polyoxyethylene Castrol oil derivatives, polyethoxylene sorbitan fatty acid esters and mixture thereof, preferably polyoxyethylene sorbitan fatty acid ester.

6. The composition according to claim 4 or 5, wherein the surfactant is present in the composition in an amount of more than 0.01 % (w/v), preferably in an amount of 0.05 to 3 % (w/v), more preferably 0.1 % to 1 % (w/v), most preferably from 0.1 % to 0.5 % (w/v).

7. The composition according to claim 4, 5 or 6, wherein the surfactant is an ester of polyoxyethylene sorbitan fatty acid, preferably polysorbate 80.

8. The composition according to any of the preceding claims, wherein the composition further comprises one or more ingredients selected form the group consisting of buffering agents, pH adjusting agents, viscosity modifying agents and tonicity agents, preferably buffering agents, tonicity agents and pH adjusting agents.

9. The composition according to claim 8, wherein the buffering agent is selected from the group consisting of phosphates, citrates, acetates and mixture thereof, preferably citric acid, phosphoric acid, sodium or potassium phosphate, dibasic sodium phosphate, sodium or potassium acetate and mixture thereof, more preferably dibasic sodium phosphate, most preferably dibasic sodium phosphate dodecahydrate.

10. The composition according to claim 8 or 9, wherein the buffering agent is present in the composition in an amount of 0.5 % to 3 % (w/v), preferably 1 % to 1.5 % (w/v), most preferably 1.26 % (w/v).

11. The composition according to claim 8, wherein the tonicity agent is selected from the group consisting of sodium chloride, mannitol, glycerin, sorbitol and mixture thereof, preferably sodium chloride.

12. The composition according to claim 8 or 11, wherein the tonicity agent is present in the composition in an amount of 0.1 % to 3 % (w/v), preferably 0.4 % to 1 % (w/v), most preferably 0.6 % (w/v).

13. The composition according to claim 8, wherein the pH adjusting agent is selected from the group consisting of hydrochloric acid, sodium hydroxide and mixture thereof.

14. The composition according to any preceding claims, wherein the composition has a pH of 4 to 8, preferably 6 to 7.5, most preferably 7.

15. The composition according to any of the preceding claims, wherein the composition consists of olopatadine or pharmaceutically acceptable salts thereof, a surfactant, a tonicity agent, a buffering agent and a pH adjusting agent.
